(19) Europäisches Patentamt

European Patent Office

Office européen des brevets

(11) **EP 1 038 505 A2**

(12) **EUROPÄISCHE PATENTANMELDUNG**

(43) Veröffentlichungstag:
27.09.2000 Patentblatt 2000/39

(51) Int. Cl.7: **A61B 18/18**

(21) Anmeldenummer: 00250088.2

(22) Anmeldetag: **09.03.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **23.03.1999 DE 19914108**

(71) Anmelder: **PlasmaPhotonics GmbH**
**12489 Berlin (DE)**

(72) Erfinder: **Conrady, Jürgen**
**13051 Berlin (DE)**

(74) Vertreter:
**Effert, Bressel und Kollegen**
**Radickestrasse 48**
**12489 Berlin (DE)**

(54) **Bestrahlungsanordnung, insbesondere zur optischen Thermolyse**

(57) Die Erfindung betrifft eine Bestrahlungsanordnung (1), insbesondere zur optischen Thermolyse, umfassend eine nichtkohärente NIR-Lichtquelle (2), einen der NIR-Lichtquelle (2) zugeordneten Lichtwellenleiter (3) und eine Kühleinrichtung (4), wobei der Lichtwellenleiter (3) einstückig aus einem massiven Saphir oder Quarz gebildet ist, um dessen Umfang mindestens teilweise ein in einem geschlossenen Kühlkreislauf befindliches Kühlmittel sich befindet.

Fig. 1

EP 1 038 505 A2

**Beschreibung**

Die Erfindung betrifft eine Strahlungsanordnung, insbesondere zur optischen Thermolyse, umfassend eine nichtkohärente NIR-Lichtquelle, einen Lichtwellenleiter und eine Kühleinrichtung zur Kühlung der oberen Hautschichten und eine pulsbare NIR-Lichtquelle.

Der spektrale Bereich zwischen ca. 700 und 1.300 nm ist für verschiedene dermatologische und auch bildgebende Anwendungen sehr interessant, da in diesem Spektralbereich eine nur noch geringe Absorption durch den Hautfarbstoff Melanin erfolgt und somit eine große Eindringtiefe der Strahlung in diesem Wellenlängenbereich gewährleistet ist. Mögliche Lichtquellen, die diesen Spektralbereich abdecken, sind Neodym-YAG-Laser sowie GaAs-Laserdioden. Darüber hinaus finden nichtkohärente Lichtquellen, wie beispielsweise Xenon-Hochdrucklampen, Verwendung.

Obwohl die Absorption des Blutfarbstoffs im nahen Infrarot um mehrere Größenordnungen geringer ist als im sichtbaren Bereich, sind doch die Absorptionsunterschiede zum umliegenden Gewebe so stark, daß eine selektive Erwärmung von bestimmten Gefäßen durch NIR-Strahlung möglich ist. Beispielsweise beträgt die Eindringtiefe von Nd-YAG-Strahlung bei einer Wellenlänge von 106 μm in Haut 4 mm und in Blut 0,8 mm (Berlien, Müller - Angewandte Lasermedizin II - 3.1, 33, Tab.6 I, EcoMed-Verlag 1997). Bei den interessierenden Gefäßen handelt es sich beispielsweise um erworbene Gefäßmißbildungen aus dem Varikosiskomplex (Krampfadern). Die Erwärmung des Gefäßlumens, beispielsweise durch Gefäßabsorption, führt zu einer Schädigung des Gefäßendothels. Hierdurch sinken die vom Endothel abgegebenen gerinnungshemmenden Faktoren wie Stickoxide (NO) sowie Prostazyklin ($PGI_2$) ab. Thrombozyten lagern sich an der Gefäßwand an und bilden schließlich einen aus Erythrozyten und Thrombozyten bestehenden, das Gefäß verschließenden roten Thrombus. Jetzt fließt durch die Krampfader kein Blut mehr, und im weiteren Verlauf wird dieser Thrombus von Bindegewebe durchwachsen und somit das Gefäß verödet.

Bei der Behandlung oberflächlicher Gefäßveränderungen reicht es oft aus, ohne externe Kühlung auszukommen, da infolge der kurzen Impulszeiten eine gewebeschädigende Wärmeausbreitung in das umliegende Hautgewebe ausbleibt. Will man jedoch tieferliegende Strukturen, wie beispielsweise Haarwurzeln, über die kritische Temperatur von ca. 80°C erhitzen, ohne die oberen Hautschichten thermisch zu verletzen, so ist eine externe Kühlung unabdingbar, die die Absorptionswärme der Strahlung aus der obersten Hautschicht, die unverletzt bleiben soll, ableitet.

Aus der US-5,282,797 ist eine Bestrahlungseinrichtung bekannt, umfassend einen NIR-Laser, einen Lichtwellenleiter und einen mit einem Kühlmittel gefüllten Behälter. In einer bevorzugten Ausführungsform ist der Behälter als flexibler Beutel mit einem Zulauf und einem Ablauf ausgebildet und in einen geschlossenen Kreislauf eingebunden. Der flexible Beutel wird zwischen der Haut und dem Lichtwellenleiter angeordnet. Durch die flexible Ausbildung des Beutels paßt sich dieser der Umgebung der Haut an, und der Lichtwellenleiter kann direkt auf den Beutel aufgesetzt werden. Beutel und Kühlmittel sind transparent für die NIR-Strahlung ausgewählt, so daß diese nahezu ungeschwächt auf die Haut auftrifft, wobei über das Kühlmittel der oberen Hautschicht Wärme entzogen wird und somit thermische Schädigungen der oberen Hautschicht vermieden werden. Nachteilig an den bekannten Bestrahlungsanordnungen mit NIR-Lasern sind die extrem großen Kosten. Ein weiteres Problem stellen die erforderlichen ultralangen NIR-Pulse mit einer Pulslänge von beispielsweise 50 - 200 ms mit einer Pulsenergie von beispielsweise 50 - 200 J dar, die nur mit großem Aufwand zu erreichen sind. Um in mehreren mm Tiefe noch ausreichende Energiedichten zu erreichen, wurde es erforderlich, die Behandlungsfläche auf mindestens 1 cm$^2$ und die Pulslängen auf bis zu mehrere 100 ms zu erhöhen.

Diese günstigen Behandlungsparameter kommen den physikalischen Eigenschaften eines Lasers nicht entgegen. Die Pulszeiten von gütegeschalteten Lasern sind so kurz, daß nur kleinste oberflächliche Strukturen behandelt werden könnten. Für verschiedene Hauterkrankungen ist die gleichzeitige Behandlung von einem oder mehreren cm$^2$ Hautoberfläche günstig. Die erforderliche Strahl-aufweitung von beispielsweise 40.000 μm$^2$ auf 1 cm$^2$ ist mit einem erheblichen technischen Aufwand verbunden und hebt gleichzeitig den eigentlichen lichtkonzentrierten Lasereffekt teilweise auf.

Der vergleichsweise schlechte Wirkungsgrad der Laser bedingt einen erhöhten technischen Aufwand, so daß in jüngster Zeit vermehrt Laserdiodenbarren eingesetzt werden, bei denen eine Vielzahl einzelner Laserdioden von beispielsweise 1 - 3 W über Einzelfasern mit dem Einsatzort verkoppelt sind. Diodengepumpte Festkörperlaser, aber auch nichtkohärente Blitzlampen haben üblicherweise Pulslängen im ns- bis ms-Bereich, so daß aufwendige Resonatormodifikationen für längere Pulse notwendig sind. Laserdioden haben den Nachteil, daß diese aus thermischen Gründen nicht im gütegeschalteten Pulsbetrieb betrieben werden können, sondern sich lediglich im cw-Betrieb ein-und ausschalten lassen. Um die erforderliche Leistung zu erreichen, benötigt man eine sehr hohe Anzahl von Laserdiodenstacks, die zur Zeit mehrere 10.000 DM kosten.

Aus der US-5,620,478 ist eine Bestrahlungsanordnung mit einer nichtkohärenten Strahlungsquelle bekannt, die als Xenon-Hochdruckblitzlampe ausgebildet ist. Die Blitzlampe ist dabei in einem Reflektor angeordnet, dem ein Lichtwellenleiter zugeordnet ist, in den die erzeugte optische Strahlung eingekoppelt wird und dessen Austrittsfläche auf die bestrahlte Hautpartie aufgesetzt wird. Aufgrund der divergenten Strahlung der Lichtquelle ist eine Kühleinrichtung, wie dies von den Laseranordnungen bekannt ist, nicht möglich, da es an den Grenzschichten zu dem Kühlmittel zu Reflexionen und Streuungen kommen würde. Daher wird als Alternative ein für die NIR-Strahlung transparentes, kühlendes Gel vorgeschlagen, das auf die zu behandelnde Hautpartie aufgetragen wird. Wie sich jedoch in der praktischen Anwendung gezeigt hat, ist die Wärmeabfuhr durch das Gel zu gering, so daß es häufig zu Verbrennungen in der oberen Hautschicht kommt. Ein weiterer Nachteil ist, daß infolge der hohen Plasmatemperatur von 6000°C ein bis in den UVC-Bereich reichendes kontinuierliches Spektrum mit nur einem geringen NIR-Anteil erzeugt wird, so daß der Wirkungsgrad verhältnismäßig schlecht ist. Zudem sind daher aufwendige Filtermaßnahmen zur Unterdrückung der nicht gewünschten Spektralbereiche notwendig. Weiterhin benötigen die Blitzlampen aufgrund der hohen Temperaturen eine aufwendige Wasserkühlung, die Service- und Herstellkosten weiter erhöhen.

Aus der US-5,344,418 ist eine Bestrahlungsanordnung mit einer Hochdruckentladungslampe als NIR-Lichtquelle bekannt, deren emittierte Strahlung in einem Lichtwellenleiter eingekoppelt wird, über eine erste Linse gesammelt, über einen Spiegel umgelenkt, eine zweite Linse gesammelt und über eine weitere aus Saphir bestehende Linse aus der Anordnung heraustritt. Die Saphirlinse wird dabei von einem Kühlgas wie $CO_2$, Freon oder einem ähnlichen Gas umspült und entsprechend abgekühlt, wobei dann die gekühlte Saphirlinse direkt auf die zu behandelnde Hautpartie aufgesetzt wird. In einer alternativen Ausführungsform wird vorgeschlagen, daß das Licht aus dem Lichtwellenleiter direkt auf die Saphirlinse gestrahlt wird. Nachteilig an der bekannten Vorrichtung ist die geringe Wärmekapazität der Saphirlinse aufgrund des geringen Volumens. Verstärkt wird dieses Problem durch die Beschränkung auf Kühlgase, die im Vergleich zur Kühlflüssigkeit erheblich weniger Wärme aufnehmen können. Flüssigkeiten können wegen der ansonsten auftretenden Dispersion an der Saphirlinse nicht verwendet werden. Ein weiteres Problem stellen thermisch isolierende Dampfschichten des Kühlgases oberhalb der Saphirlinse dar, die durch geeignete Verwirbelungstechnik vermieden werden müssen.

Aus der US-5,830,208 ist eine Bestrahlungsanordnung bekannt, bei der die Kühleinrichtung als Peltierelement ausgebildet ist. Der eine Nachteil der bekannten Anordnung ist, daß die handelsüblichen Peltierelemente nicht ausreichend Kühlleistung zur Verfügung stellen, um Verbrennungen der oberen Hautschicht zu verhindern. Bei einem Einsatz der Peltierelemente in einer Anordnung gemäß der US-5,344,418 stellt sich zusätzlich das Problem, daß eine seitliche Kühlung der Saphirlinse aufgrund der geringen Umfangsfläche der Linse nur ungenügend ist. Verstärkt wird dieser Effekt durch die notwendige seitliche Fassung der Linse, die wiederum einen erhöhten Wärmeübergangswiderstand bildet. Zudem führen geringe Luftspalte zwischen der Fassung und der Saphirlinse zu einer bereits erheblich ins Gewicht fallenden thermischen Isolierung der Saphirlinse.

Aus der US-5,814,040 ist eine Bestrahlungsanordnung mit einem NIR-Laser bekannt, der eine Sprüheinrichtung für ein Kältemittel, vorzugsweise R 134 A zugeordnet ist, mittels derer die zu behandelnde Hautoberfläche kurzzeitig im Bereich von 10 ms besprüht und somit gekühlt wird. Dadurch sollen einerseits die erwähnten Verbrennungen in der oberen Hautschicht vermieden werden und andererseits sichergestellt werden, daß die tieferliegenden, zu erwärmenden Hautschichten nicht abgekühlt werden. Zwar führt die entstehende Verdunstungswärme zu einem schnellen Abkühlen des Gewebes, jedoch ist die Abkühlung nur schwer steuerbar, so daß zum einen nur sehr kurze Impulse im Bereich von 100 ms verwendet werden und zum anderen durch starkes Anblasen vermieden werden muß, daß sich eine thermisch isolierende Kühlmittelschicht ausbildet. Unter ökologischen Gesichtspunkten ist weiterhin das Entweichen von für den Abbau der Ozonschicht verantwortlichen FCKW's bedenklich. Ein weiteres Problem stellt bei Verwendung nichtkohärenter Strahlungsquellen die Unterbrechung des Strahlenganges dar. Zwischen Haut und Applikatorende muß ein Luftspalt verbleiben, der für die Zufuhr von Kühlmittel benötigt wird. Dadurch entstehen jedoch zusätzliche Grenzschichten, die bei inkohärenten Strahlungsquellen zu erheblichen Einkoppelungsverlusten führen.

Aus der US-4,233,493 ist eine Bestrahlungsanordnung zum Stoppen von Blutungen bei chirurgischen Eingriffen bekannt, umfassend eine als Glühleuchte ausgebildete NIR-Lichtquelle, einen die IR-Lichtquelle umgebenden Reflektor, einen Lichtwellenleiter und eine an der Austrittsöffnung des Lichtwellenleiters angeordnete Kappe oder Scheibe, die beispielsweise aus Quarz oder Saphir besteht. Die Glühleuchte ist dabei vorzugsweise als konventionelle Wolfram-Glühleuchte ausgebildet.

Der Erfindung liegt daher das technische Problem zugrunde, eine Bestrahlungsanordnung zur optischen Thermolyse zu schaffen, mit der unter Einsatz einer inkohärenten NIR-Lichtquelle eine ausreichende Kühlleistung zur Verhinderung von Verbrennungen in den oberen Hautschichten verfügbar ist. Ein weiteres technisches Problem ist die Schaffung einer preiswerten und kompakten pulsbaren NIR-Lichtquelle.

Die Lösung des technischen Problems ergibt sich durch die Merkmale der Patentansprüche 1, 6 und 7. Weitere vorteilhafte Ausgestaltungen der Erfindung ergeben sich aus den Unteransprüchen.

Durch die einstückige Ausbildung des Lichtwellenleiters aus einem massiven Quarz oder Saphir, dessen Umfang mindestens teilweise von einem in einem geschlossenen Kühlkreislauf befindlichen Kühlmittel umströmt wird, stellt der Lichtwellenleiter selbst eine große Wärmesenke dar, so daß es beim direkten Aufsetzen des Lichtwellenleiters auf die Hautoberfläche zu keinen Verbrennungen kommt, da die Absorptionswärme der oberen Hautschichten über eine direkte Wärmeleitung abgeführt wird. Ein weiterer Vorteil der Anordnung ist, daß durch die seitliche Kühlung sich kein Kühlmittel im Strahlengang der NIR-Lichtquelle befindet. Ein weiterer Vorteil der Anordnung ist, daß der Lichtwellenleiter auch in der Lage ist, rückgestreute Wärmestrahlung bis zu einer Wellenlänge von 5μm wieder einzukoppeln und abzuführen.

Vorzugsweise wird als Kühlmittel eine Kühlflüssigkeit verwendet, da diese erheblich mehr Wärme im Vergleich zu gasförmigen Kühlmitteln abführen können. Prinzipiell können alle bekannten Kühlflüssigkeiten verwendet werden, wie beispielsweise Wasser, Alkohol oder Glykol. Der Brechzahlunterschied zwischen Saphir bzw. Quarz zu den verwendeten Kühlmitteln ist dabei derart groß, daß fast eine vollständige Totalreflexion im Lichtwellenleiter auftritt. Der Strahlungsweg wird ähnlich wie bei einer kleinen Lichtleitfaser bis zum Ort des des Hautkontaktes nicht unterbrochen, so daß am Austrittsort gut durchmischtes Licht mit einer Apertur aus dem Verhältnis der beiden Brechzahlen austritt.

In einer weiteren bevorzugten Ausführungsform ist der Lichtwellenleiter zylinderförmig oder rechteckförmig ausgebildet. Die zylinderförmige Ausführungsform läßt sich sehr einfach herstellen und mittels O-Ringen abdichten, wobei der Durchmesser des Zylinders beispielsweise 12mm bei einer Länge von 70 mm beträgt. Der Vorteil einer rechteckförmigen Ausbildung von beispielsweise 8mm X 35mm ist, daß längere Gefäße simultan in ihrem Gesamtverlauf erwärmbar sind, so daß eine Wirkungsverminderung durch die Eigenkühlung des Gefäßes reduziert wird.

In einer weiteren bevorzugten Ausführungsform wird das langweilige Infrarot unterdrückt. Hierzu gibt es prinzipiell verschiedene Möglichkeiten. Im einfachsten Fall wird zwischen der NIR-Lichtquelle und der Stirnseite des Lichtwellenleiters ein entsprechender Filter angeordnet, der diesen Spektralbereich unterdrückt. Allerdings stellt die thermische Belastung der Filter ein Problem dar. Daher wird vorzugsweise der langweilige Infrarotfilter der das Applikatorende bildenden Stirnseite zugeordnet, wo dieser beispielsweise mittels eines geeigneten thermischen und optischen Kitts mit der Stirnseite verbunden wird.

In einer alternativen Ausführungsform wird ausgenutzt, daß die Kühlflüssigkeiten, insbesondere Wasser sehr gute langweilige IR-Filter darstellen. Insbesondere Wasser filtert exakt die Spektralbereiche heraus, die ansonsten zu einer unerwünschten Eiweißkoagulation an der Hautoberfläche führen könnten. Daher wird die der NIR-Lichtquelle zugeordnete Stirnseite vollständig innerhalb der Kühlflüssigkeit angeordnet. Zwischen der NIR-Lichtquelle und der Stirnseite wird dann der Kühlkreislauf mittels eines Quarz-oder Saphirfensters verschlossen. Die zuvor erwähnten Probleme beim Durchgang inkohärenter Strahlung durch eine Flüssigkeitsschicht können durch eine entsprechend große Apertur der NIR-Lichtquelle gelöst werden, was später noch näher erläutert wird.

Eine weitere Möglichkeit zur Unterdrückung der langwelligen Infrarotstrahlung besteht darin, beispielsweise die NIR-Lichtquelle in einem Brennpunkt eines Ellipsoid-Reflektors anzuordnen, in dessen anderem Brennpunkt die Stirnseite des Lichtwellenleiters angeordnet ist. Der Reflektor besteht aus einem langweilige Infrarotstrahlung transmittierenden Material wie beispielsweise Teflon. Zur Verhinderung einer direkten Einkopplung von der NIR-Lichtquelle in den Lichtwellenleiter ist im direkten Strahlengang ein Filter oder ein weiterer Reflektor angeordnet. Der Vorteil dieser Anordnung ist, daß die NIR-Nutzstrahlung nicht eine Flüssigkeitsschicht passieren muß, jedoch verliert die Bestrahlungsanordnung aufgrund des Volumens des Ellipsoid-Reflektors etwas von seiner Kompaktheit.

Eine weitere bevorzugte Anwendung bezieht sich auf den Einsatz der Anordnung zu einer gewebeschonenden Kryotherapie. Üblicherweise wird die Kryotherapie mit einem Metallapplikator durchgeführt, der von innen entweder mit einem Kühlmittel, wie beispielsweise R 134 A, oder direkt mit flüssigem Stickstoff durchströmt wird. Setzt man einen derartig vorgekühlten Applikator auf die zu behandelnde Läsion, wie beispielsweise ein oberflächliches Karzinom, auf, so kommt es entsprechend der gewebetypischen Wärmeleitkonstante und dem Wärmeübergang zwischen Gewebe und Applikator zu einer Kälteausbreitung im Gewebe. Diese Kälteausbreitung erfolgt in annähernd konzentrischen Isothermen, wobei vor allem größere, schnell fließende Gefäße das isothermische Ausbreitungsprofil deformieren.

Der erfrierungsbedingte Gewebeschaden wird durch eisbedingte, biophysikalische Gewebeveränderungen verursacht. Insbesondere intrazellulärem Eis kommt über ein direktes Zerreißen von Zellmembranstrukturen eine besonders schädliche Rolle zu. Die Entstehung von physikalisch wirksamem, intrazellulärem Eis kann nun durch bestimmte Maßnahmen verhindert werden. Hierzu gehören die Induktion von Scherkräften mit Hilfe von Ultraschall, Magnetfeldern oder photoakustischen Verfahren. Darüber hinaus ist es mit Hilfe einer gepulsten, zeitlich synchronisierten Bestrahlung der zu schützenden Hautoberfläche möglich, den Grad des "undercooling" erheblich anzuheben. Hierunter versteht man die Absenkung der Temperatur unterhalb des Schmelzpunktes, wobei durch Abwesenheit von Kristallisationskeimen die gewebeschädigende Eisbildung erheblich verhindert werden kann.

Mit der Anordnung ist es somit möglich, tiefere Gewebsstrukturen einer Kryotherapie zu unterziehen und gleichzeitig durch eine Beeinflußung der oberen Gewebeschichten mit Hilfe von optischer, magnetischer und akustischer Energie deren Schädigung zu verhindern. Die bevorzugte Anwendung erlaubt nun erstmals auch die kryotherapeutische Behandlung von tieferen Gewebsstrukturen, wie beispielsweise Haarwurzeln oder Gefäßanomalien. Ein weiterer möglicher Vorteil der Kryotherapie ist ihre gewebsschonende Anwendung.

Im Gegensatz zu einer Kryotherapie kommt es bei einer Photokoagulation immer zu einer Proteindenaturierung. Dies löst in der Regel immer eine starke Entzündungsreaktion aus, in dessen Rahmen die beteiligten Entzündungszellen sekundäre Schäden verursachen. Hierzu gehören beispielsweise Narben und Hyperpigmentierungen. Wird die erwünschte Gewebenekrose nicht durch Hitzeeinwirkung sondern durch Kälteeinwirkung erzeugt, so ist die hierbei auftretende Nekrose mit einer wesentlich kleineren Entzündungsreaktion verbunden, da es zwar durch Mikrokristallbildung in den Einzelzellen zu einer Perforation der Zellwand, einen Austritt von Intrazellulärflüssigkeit und nachfolgendem Zelltod kommt, jedoch treten in diesem Fall zu keiner Zeit denaturierte Eiweiße auf. Kryotherapie wurde bisher ausschließlich mit Hilfe von Applikatoren durchgeführt, die eine Schonung der oberen Gewebsschichten nicht zulassen.

Die Bestrahlungsanordnung kann neben der bereits erwähnten optischen Thermolyse auch zur Behandlung von Hämorrhoiden, der lokalisierten Form der Schuppenflechte (Plaque), NIR-Bindegewebsinteraktion (Zellulite) sowie einiger Formen des Prostatahypertrophie-Symtomenkomplexes verwendet werden. Bei einer Senkung der Bestrahlungsstärke kann die Bestrahlungsanordnung bei chronisch rezidivierenden Entzündungen im Nasennebenhöhlen- und Stirnhöhlenbereich eingesetzt werden, wo der schleimlösende Einfluß derartiger Wärmebehandlungen ausgenutzt wird. Ein weiteres Anwendungsfeld ist die günstige Beeinflußung von kollagensynthetisierenden Fibroblasten, bei denen durch NIR eine Änderung des Kollagensynthesemusters erreicht werden kann. Hiermit ist sowohl eine positive Beeinflussung von alterungsbedingten Hautfalten als auch der bindegewebsbedingten ungleichmäßigen Fettzellenanordnung (Zellulite) möglich.

In einer weiteren bevorzugten Ausführungsform umfaßt die NIR-Lichtquelle eine Wolfram-Nacktwendel, einen halboffenen Reflektor, eine Schutzgasquelle und eine Spannungspulse erzeugende Spannungsquelle, wobei die Wolfram-Nacktwendel einseitig im Reflektor gesockelt und mit der Spannungsquelle verbunden ist, der Lichtwellenleiter in den halboffenen Reflektor hineinragt, so daß zwischen Lichtwellenleiter und Reflektor eine Öffnung definiert wird, über die Schutzgas in den Reflektor ein- und/oder ableitbar ist. Vorzugsweise ist der Reflektor als Keramikreflektor ausgebildet und derart dimensioniert, daß dieser direkt auf den Lichtwellenleiter aufgesetzt werden kann. Hierbei kommt es nicht zu einem luftdichten Abschluß, sondern es verbleibt entweder ein umlaufender Randspalt, oder es wird über eine oder mehrere kleine Kerben eine Undichtigkeit erzeugt. Über eine externe Zuleitung, beispielsweise eine feine Bohrung in der Nähe des Wendelsockels, wird dann das Schutzgas eingeleitet und kann über die Öffnungen zwischen Lichtwellenleiter und Reflektor entweichen. Dadurch entsteht eine äußerst kompakte Anordnung, bei der der Abstand zwischen der emittierenden Wolfram-Nacktwendel und dem Lichtwellenleiter sehr gering ist, so daß die Einkoppelverluste ebenfalls sehr gering sind. Des weiteren erlaubt diese Anordnung ein einfaches Auswechseln von defekten Wendeln, sowie den problemlosen Einsatz verschiedener Wendelgeometrien, wobei vorzugsweise Flachwendeln zur Anwendung kommen. Ein weiterer Vorteil dieser Anordnung ist, daß diese vollkommen drucklos aufgebaut ist, so daß keinerlei Explosionsgefahr besteht. Im Falle eines Versagens der Schutzgaszufuhr verglüht lediglich die Wendel in wenigen Millisekunden, und das Gerät stellt seine Funktion ein. Im Gegensatz zu gepulsten Gasentladungslampen entfallen bei dieser Anordnung Hochspannungsbauteile. Bei Verwendung von Niedervoltwendeln und der Bereitstellung entsprechend dimensionierter Kupferzuleitungen kann die Lichtquelle beispielsweise mit 48 V gepulst werden, wodurch die Patientensicherheit weiter erhöht wird. Ein weiterer Vorteil des Schutzgasstromes ist, daß die Glühwendel stärker im Überlastbereich betrieben werden kann, ohne daß es zu einem Aufschmelzen kommt, da der Schutzgasstrom als Kühlmittel für die Wendel wirkt. Des weiteren ist es möglich, die Oberfläche der Glühwendel mit einer geeigneten Mikrostrukturierung auszubilden, um durch Interferenzeffekte das abgestrahlte Spektrum zu beeinflußen. Da die Strukturierung von Mehrfachwendeln relativ aufwendig ist, kann daher in einer weiteren bevorzugten Ausführungsform eine Wolframfolie verwendet werden, wie sie aus Bandlampen bekannt ist. Diese Wolframfolie kann dann beispielsweise durch chemische Bedampfungsverfahren oder Ionenätzung mikrostrukturiert werden.

Alternativ kann die pulsbare NIR-Lichtquelle durch eine GlühwendelHalogenlampe realisiert werden, die beispielsweise von einer Kondensatorbatterie mit einem rechteckförmigen Überspannungspuls von beispielsweise 500 V über 20 - 200 ms betrieben wird. Trotz der extremen Stromstärken von mehreren 10 A kommt es nicht zu einem Durchglühen der Wendel, sondern zu einer überraschend extremen Verkürzung der Anstiegszeit in eine Größenordnung von 7 ms (Vergleich: Wird eine handelsübliche Glühwendel-Halogenlampe ans Netz geschaltet, so ist die Anstiegszeit ungefähr 200 - 300 ms). Durch die extreme Kurzzeitüberlastung kommt es darüber hinaus zu einer Erhöhung der Wendeltemperatur auf über 3000°C und hiermit zu einer Verschiebung des Strahlungsmaximums zu kleineren Wellenlängen. Bei einer bevorzugten Ausführung der Glühwendel aus Wolfram verschiebt sich das Maximum von 1000 nm auf 870 nm. Neben einer besseren Ausnutzung des sogenannten optischen Fensters der Haut, ist auch die Flächenleistung der Glühwendel in Folge ihrer höheren Temperatur und der damit verbundene Wirkungsgrad signifikant erhöht. Ein weiterer Vorteil dieser Betriebsart bezieht sich auf die Beeinflussung der Wolframwendelstruktur selbst. Im Herstellungsprozeß wird ein fibröses Drahtmaterial verwendet, das eine ausreichende Duktilität für die Biegungsvorgänge besitzt. Für einen befriedigenden Lampenbetrieb ist die Umsetzung in eine kristalline Struktur günstig. Beispielsweise wird in der US-4,012,659 und der US-4,020,383 beschrieben, daß elektrische Pulse die Lebensdauer von normalen Glühlampen günstig beeinflussen. Neben der Wolframrekristallisierung wird erwähnt, daß mit Hilfe der Pulse Kontaktprobleme, beispielsweise aufgrund von Oxidation, zwischen der Drahtaufhängung und der Wolframwendel beseitigt werden können.

Eine weitere Möglichkeit, die Abstrahlleistung weiter zu erhöhen, ist eine möglichst enge Ummantelung der Halogenlampe durch einen Reflektor, der lediglich im Auskoppelbereich geöffnet ist, wobei der Reflektor beispielsweise als polierter Metallreflektor ausgebildet ist. Hierdurch kommt es zu einer Reabsorption der reflektierten Strahlung durch die Wendel und damit zu einer weiteren Erhöhung des Wirkungsgrades im Nutzstrahlbereich.

Die Erfindung wird nachfolgend anhand eines bevorzugten Ausführungsbeispieles näher erläutert. Die Fig. zeigen:

Fig.1    eine Prinzipdarstellung einer Bestrahlungsanordnung zur optischen Thermolyse,

Fig.2    einen Längsschnitt durch den Lichtwellenleiter,

Fig.3    eine Prinzipdarstellung der Bestrahlungsanordnung mit unterdrückter langwelliger Infrarotstrahlung und

Fig.4    eine Prinzipdarstellung einer Bestrahlungsanordnung für die Kryotherapie.

Die Bestrahlungsanordnung 1 zur optischen Thermolyse umfaßt eine pulsbare NIR-Lichtquelle 2, einen Lichtwellenleiter 3 und eine Kühleinrichtung 4. Die Kühleinrichtung 4 umfaßt neben einem Sammelgefäß für die Kühlfüssigkeit 9 einen Zulauf 5, einen Abfluß 6 und einen zylinderschalenförmigen Bereich 7, der den Lichtwellenleiter 3 nahezu vollständig umgibt. Der zylinderschalenförmige Bereich 7 ist mittels O-Ringen 8 hermetisch dicht um den Lichtwellenleiter 3 herum angeordnet, so daß sich ein geschlossener Kühlkreislauf ergibt. Der Lichtwellenleiter 3 ist als massiver Zylinder aus Saphir oder Quarz ausgebildet, dessen der NIR-Lichtquelle 2 zugewandte Stirnfläche 10 möglichst dicht der NIR-Lichtquelle 2 zugeordnet ist. Die gegenüberliegende Stirnseite 11 bildet das Applikatorende, das direkt auf einen zu behandelnden Hautbereich 12 aufgesetzt wird. Die NIR-Lichtquelle 2 ist als Glühwendel-Halogenlampe mit einer Glühwendel 13 aus Wolfram ausgebildet, die mittels einer nicht dargestellten Spannungsquelle im Überlastbereich angesteuert wird. Um den Quarzkolben der Glühwendel-Halogenlampe ist ein Reflektor angeordnet, der nur im Bereich der Stirnfläche 10 des Lichtwellenleiters 3 offen ist. Die von der NIR-Lichtquelle 2 emittierte NIR-Strahlung wird in die Stirnseite 10 eingekoppelt und tritt an der Stirnseite 11 aus. Die austretende NIR-Strahlung dringt dann in die Haut 12 ein und wird von den Gefäßen absorbiert. Der fast vollständig von Kühlflüssigkeit 9 umströmte Lichtwellenleiter 3 stellt dann eine sehr große Wärmesenke für die oberste Hautschicht dar und verhindert so Verbrennungen aufgrund zu großer Wärmeentwicklung. Somit übernimmt der Lichtwellenleiter 3 in der Bestrahlungsanordnung 1 eine Doppelfunktion, nämlich die des optischen Leiters und die eines Kühlelementes. Daher muß das Material sowohl ausreichend gute optische als auch thermische Eigenschaften hinsichtlich der Wärmeleitfähigkeit aufweisen. Diese beiden Eigenschaften werden sehr gut von Saphir, Quarz und Diamant erfüllt, wobei letzterer zwar physikalisch am geeignetsten ist, jedoch auch am teuersten ist, so daß zumindest derzeit aus kostentechnischen Grün-den eine Verwendung nicht in Frage kommt. Prinzipiell kämen aber Kunststoffe in Frage, die die beiden notwendigen physikalischen Eigenschaften miteinander vereinen.

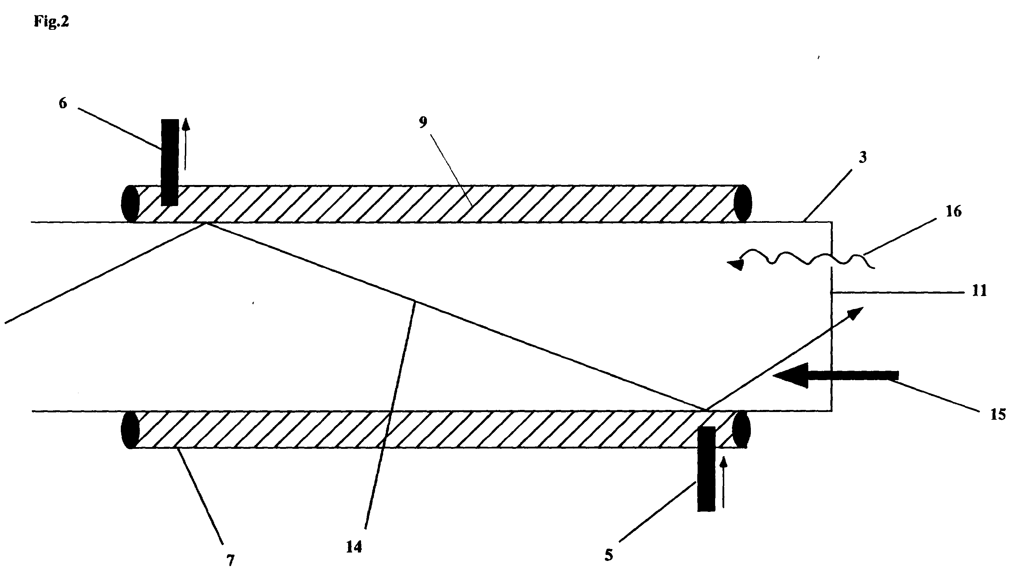

In der Fig.2 ist der Lichtwellenleiter 3 im Längsschnitt dargestellt. Die NIR-Nutzstrahlung 14 bewegt sich mittels Totalreflexion im Lichtwellenleiter 3 fort, wenn der Auftrittswinkel an der Grenzfläche zur Kühlflüssigkeit 9 größer als ein Grenzwinkel ist. Dieser von den Brechzahlen abhängige Grenzwinkel liegt für Saphir und die üblichen Kühlflüssigkeiten bei ca. 30°, so daß die Ausbreitungsverluste äußerst gering sind. Aufgrund des Temperaturgradienten an der Haut 12 kommt es zu einem direkten Wärmestrom 15 von der Haut 12 zum Lichtwellenleiter 3, wo die Wärme über die Kühlflüssigkeit 9 abgeführt wird. Des weiteren wird von der Haut 12 reflektierte Wärmestrahlung 16 in die Stirnseite 11 des Lichtwellenleiters 3 eingekoppelt und ebenfalls abgeführt.

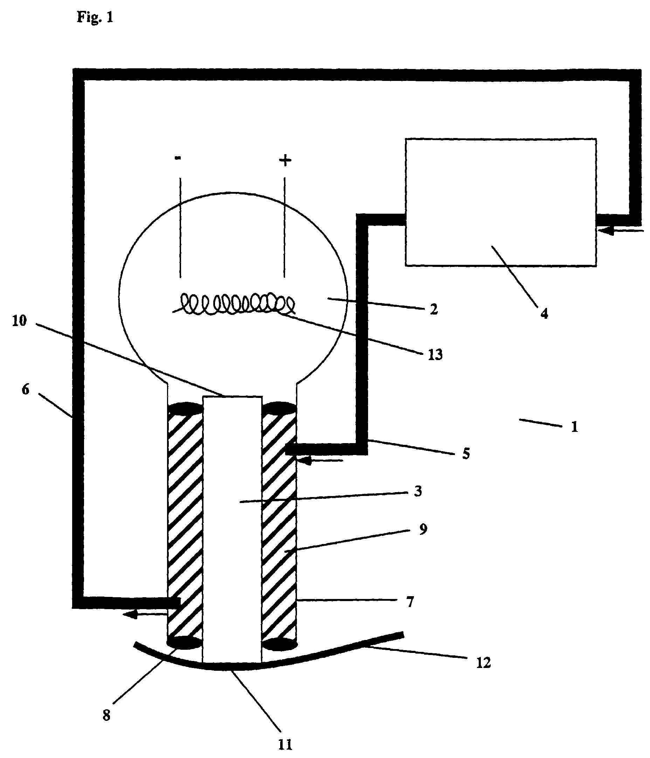

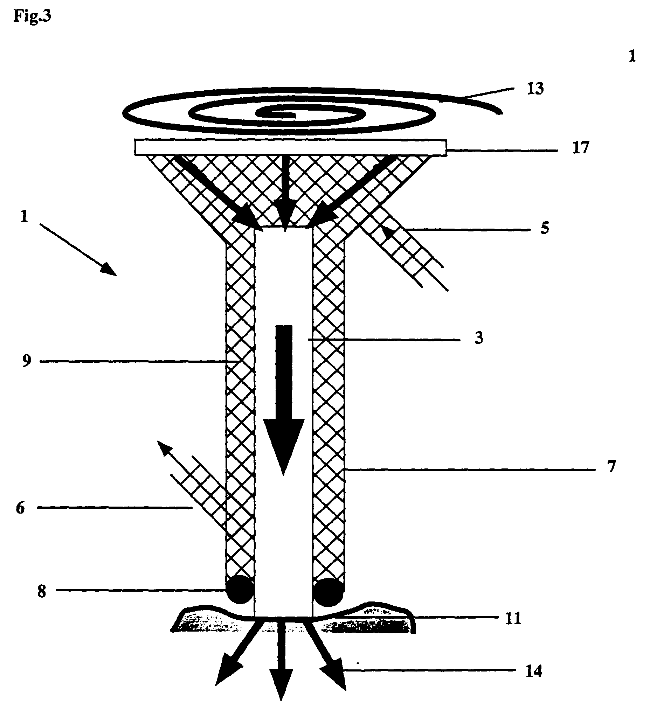

In der Fig.3 ist eine Bestrahlungsanordnung 1 mit Unterdrückung langwelliger Infrarotstrahlung dargestellt. Der wesentliche Unterschied zur Bestrahlungsanordnung 1 gemäß Fig.1 besteht darin, daß die der NIR-Lichtquelle 2 zugeordnete Stirnseite 10 des Lichtwellenleiters 3 sich vollständig innerhalb der Kühlflüssigkeit 9 befindet. Zwischen der NIR-Lichtquelle 2 und der Stirnseite 10 ist der Kühlkreislauf mittels eines Quarz- oder Saphirfensters 17 abgeschlossen. Die Apertur der NIR-Lichtquelle 2 und des Quarz- oder Saphirfensters 17 ist dabei wesentlich größer als die der Stirnseite 10 des Lichtwellenleiters 3. Bei diesem Aufbau sind folgende Vorüberlegungen von Interesse.

Jeder Lichtwellenleiter 3 oder jede Lichtleitfaser weist einen Öffnungswinkel auf. Strahlung, die innerhalb dieses Winkels auf den Lichtwellenleiter trifft, wird eingekoppelt und mittels Totalreflexion weitergeleitet. Strahlung außerhalb dieses Winkels geht durch Reflexion beim Einkoppeln bzw. als Transmissionsverlust im Lichtwellenleiter verloren. Der Öffnungswinkel ist abhängig von der Differenz der Brechungungsindices. Bei Luft als Umgebung gilt:

$$\sin i = (n^2_{Kern} - n^2_{Mantel})^{1/2},$$

wobei i der Akzeptanzwinkel der Faser ist. Befindet sich der Lichtwellenleiter 3 ohne separaten Mantel an der Luft, so ist der Akzeptanzwinkel 90° für alle Materialien mit einem Brechungsindex größer $2^{1/2}$, so daß die gesamte Strahlung aus dem Halbraum in die Faser eingekoppelt und weitergeleitet wird. Da der Brechungsindex von Saphir 1,77 beträgt, existiert ein Akzeptanzwinkel von 90° für alle Mantelmaterialien mit einem Brechungsindex kleiner 1,46 (z.B. Wasser). Aufgrund der winkelabhängigen Reflexion treten erhebliche Reflexionsverluste bei Einfallswinkeln größer 70° auf. Aufgrund dieser Tatsache können die Einkoppel-Verluste durch den Durchgang durch die wasserhaltige Kühlflüssigkeit 9 durch eine entsprechend vergrößerte Apertur der Glühwendel 13 bzw. der Bandwendel kompensiert werden.

Das Quarzfenster 17 ist beispielsweise 2 mm dick und der Abstand zwischen der Innenseite des Quarzfensters 17 zur Stirnfläche 10 des Lichtwellenleiters 3 ca. 4 mm. Daraus ergibt sich bei einem Durchmesser des Lichtwellenleiters 3 von 13 mm ein optimaler Durchmesser der NIR-Lichtquelle 2 von 24 mm. Die Ausbildung des Fensters 17 aus Quarz hat gegenüber Saphir neben dem Vorteil der geringeren Kosten den weiteren Vorteil, daß das Quarz bereits einen Teil des langwelligen infrarotlichtes absorbiert. Die restliche langwellige infrarotstrahlung wird dann von der Kühlflüssigkeit 9 absorbiert. Zur Unterdrückung möglicher Anteile der Nutzstrahlung im sichtbaren Bereich können diese durch geeignete Farbstoffe in der Kühlflüssigkeit herausgefiltert werden.

In einer alternativen Ausführungsform wird das Quarzfenster 17 und die darunterliegende Flüssigkeitsschicht durch einen Konus aus optisch transparentem Material ersetzt. Als Material kommen insbesondere Quarz oder BK7-Glas in Frage. Die Stirnflächen des Konus sind vorzugsweise plangeschliffen und poliert. Der lampennahe Durchmesser der ersten Stirnfläche beträgt beispielsweise 30 mm, wohingegen der lampenferne Durchmesser ca. 12 mm beträgt. Der Konuswinkel liegt dabei zwischen 10-15°. Vorzugsweise ist der gesamte Konus von einem infrarotreflektierendem Trichter umgeben, der vorzugsweise durch eine Goldschicht gebildet wird. Zwischen Trichter und Konus bleibt ein Randspalt erhalten, um einerseits die Totalreflexion zwischen Konusmaterial und Luft auszunutzen. Andererseits wirft der vergoldete Trichter Lichtstrahlen zurück in das optische System, die den Grenzwinkel für die Totalreflexion überschritten hatten. Die optische Kopplung zwischen der lampenfernen Stirnfläche des Konus und des Lichtwellenleiters 3 erfolgt vorzugsweise durch Silikonöl im Kapillarspalt beider Stirnflächen. Der Lichtwellenleiter 3 ist vorzugsweise als Saphirstab ausgebildet und wird teilweise im Mantelbereich durch eine Flüssigkeitsperfusion gekühlt. In Abhängigkeit von der Infrarotabsorption des Perfusionskühlmittels kann es vorteilhaft sein, den Saphirzylinder im Bereich des Flüssigkeitsmantels zu vergolden, da der Flüssigkeitsmantel im Vergleich zu einem Luftmantel den Grenzwinkel für diei Totalreflexion absenkt. Alternativ zur Goldschicht auf dem Saphirzylinder kann dieser auch in einer Hülse aus IR-reflektivem Material geführt werden. Patientenseitig ragt der Sapir ca. 2 bis 3 cm aus der Fassung, um eine möglichst hohe optische Transparenz der Behandlungsfläche zu gewährleisten. Die mittel- und langweilige IR-Absorption wird entweder durch den Werkstoff des Konus, wie beispielsweise BK7-Glas, oder mittels Filtern erreicht, wobei die Filter vorzugsweise zwischen Konus und Saphirzylinder angeordnet werden.

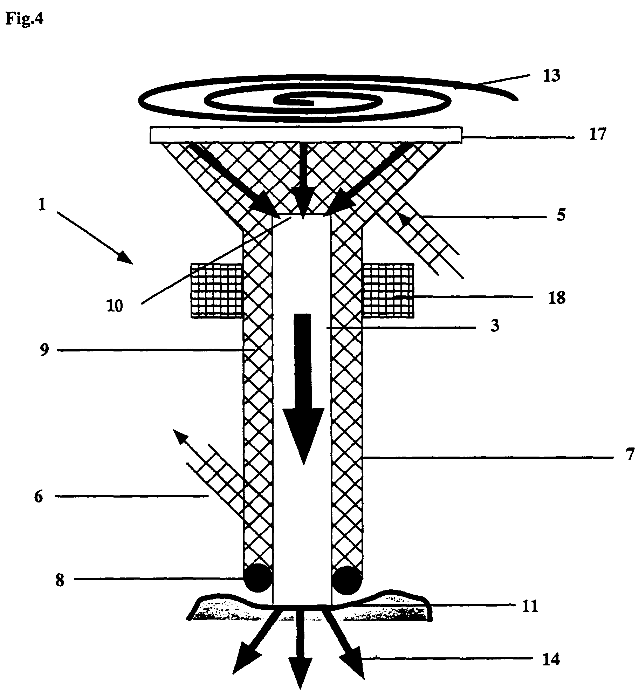

In der Fig.4 ist eine Bestrahlungsanordnung 1 für die Kryotherapie dargestellt, die in ihrem Aufbau der Bestrahlungsanordnung 1 gemäß Fig. 3 entspricht. Zusätzlich weist die Bestrahlungsanordnung 1 einen piezokeramischen Ultraschalltransducer 18 auf, der eine ungewollte Eiskristallbildung verhindern soll. Diese Betriebsart unterscheidet sich von der vorher genannten optischen Thermolyse dadurch, daß die erwünschten Gewebseinwirkungen, wie beispielsweise Thrombosierung von Krampfadern, nicht in einer Wärmezufuhr sondern in einem Wärmeentzug bestehen. Deshalb ist es erforderlich, die Temperatur des Kühlmittelapplikators so weit wie möglich zu senken, wozu als Kühlflüssigkeit 9 vorzugsweise flüssiger Stickstoff verwendet wird. Die optische Energie der NIR-Lichtquelle 2 wird in diesem Fall lediglich für die Erwärmung der obersten Hautschichten verwendet. Die hierfür erforderliche Energie von beispielsweise 5 - 20 J ist wesentlich geringer als für die direkte Photokoagulation von beispielsweise 100 - 300 J. In Abhängigkeit von der gewebstypischen Anzahl an Kristallkeimpunkten sowie deren Wachstumsverhalten ist es sinnvoll, eine hierauf abgestimmte gepulste Bestrahlung durchzuführen. Hierdurch wird in den oberen Gewebsschichten ein zyklischer Temperaturverlauf erzeugt, der die Entstehung von intrazellulärem Eis verhindert. Hierdurch kann die Temperatur zum Teil weit unter den Schmelzpunkt der Gewebeflüssigkeit abgesenkt werden, ohne daß es zu einer Eisbildung im Gewebe kommt. Die durch den Ultraschall-Transducer 18 erzeugte Ultraschallenergie verursacht darüber hinaus Scherkräfte, die der Eisbildung zusätzlich entgegenwirken.

Das Gewebe wird in diesem sogenannten Zustand des "supercooling" in keiner Weise geschädigt, da lediglich alle biochemischen Prozesse reversibel stark verlangsamt werden, ohne daß es zu einem mechanischen Zell- oder Gewebeschaden kommt. Für die Gefrierwirkung auf das tieferliegende Gewebe ist der mittlere Temperaturgradient der Geweboberfläche zum tiefen Gewebe entscheidend. Da Temperaturen von unterhalb ca. -40°C ausreichen, irreversible Gewebeschäden zu erzeugen, muß die Temperatur des Kühlmittelapplikators so tief gewählt werden, daß unter Berücksichtigung des zu erreichenden supercooling-Zustandes bzw. der zyklischen Temperaturanhebung in Gefrierpunktsnähe immer noch ein ausreichender Kältegradient vorhanden bleibt. Ein direkt mit flüssigem Stickstoff gefüllter Saphirapplikator, durch dessen Querschnitt eingestrahlte optische Energie die oberen Hautschichten bis auf 0°C kurzzeitig erwärmt, verursacht einen effektiven Temperaturgradienten von beispielsweise ca. -80 °C. Diese Temperatur ist mehr als ausreichend, um eine ausreichend schnelle Gefrierzonenausbreitung in den gewünschten Arealen zu erreichen.

**Patentansprüche**

1. Bestrahlungsanordnung, insbesondere zur optischen Thermolyse, umfassend eine nichtkohärente NIR-Lichtquelle, einen der NIR-Lichtquelle zugeordneten Lichtwellenleiter und eine Kühleinrichtung,
   **dadurch gekennzeichnet, daß**
   der Lichtwellenleiter (3) einstückig aus einem massiven Saphir oder Quarz gebildet ist, um dessen Umfang mindestens teilweise ein in einem geschlossenen Kühlkreislauf befindliches Kühlmittel sich befindet.

2. Bestrahlungsanordnung nach Anspruch 1, dadurch gekennzeichnet, daß das Kühlmittel als Kühlflüssigkeit (9) ausgebildet ist.

3. Bestrahlungsanordnung nach Anspruch 1 oder 2, dadurch gekennzeichnet, daß der Lichtwellenleiter (3) zylinder- oder quaderförmig ausgebildet ist.

4. Bestrahlungsanordnung nach einem der vorangegangenen Ansprüche, dadurch gekennzeichnet, daß der Stirnseite (11) des Lichtwellenleiters (3) ein langwelliger Infrarotfilter zugeordnet ist.

5. Bestrahlungsanordnung nach einem der Ansprüche 2 oder 3, dadurch gekennzeichnet, daß die Stirnseite (10) des Lichtwellenleiters (3) innerhalb der Kühlflüssigkeit (9) angeordnet ist und der Kühlkreislauf im Bereich zwischen der NIR-Lichtquelle (2) und der Stirnseite (10) des Lichtwellenleiters (3) mittels eines Quarz- oder Saphirfensters geschlossen ist.

6. Bestrahlungsanordnung nach einem der Ansprüche 2 bis 4, dadurch gekennzeichnet, daß zwischen der NIR-Lichtquelle (2) und dem Lichtwellenleiter (3) ein Konus aus optisch transparentem Material angeordnet ist, dessen kleinerer Durchmesser dem Lichtwellenleiter (3) zugeordnet ist.

7. Bestrahlungsanordnung nach Anspruch 6 , dadurch gekennzeichnet, daß der Konus aus Quarz oder BK7-Glas besteht.

8. Bestrahlungsanordnung nach Anspruch 6 oder 7, dadurch gekennzeichnet, daß um den Konus ein infrarotreflektierender Trichter angeordnet ist.

9. Bestrahlungsanordnung nach einem der Ansprüche 2 bis 8, dadurch gekennzeichnet, daß der Lichtwellenleiter im Bereich der Flüssigkeitskühlung mit einer infrarotreflektierenden Schicht beschichtet oder in einer infrarotreflektierenden Hülse geführt ist.

10. Pulsbare NIR-Lichtquelle zur Erzeugung von Pulslängen größer 20ms, insbesondere zur Verwendung in einer Bestrahlungsanordnung nach einem der vorangegangenen Ansprüche, dadurch gekennzeichnet, daß die NIR-Lichtquelle (2) eine Wolfram-Nacktwendel, einen halboffenen Reflektor, einen Lichtwellenleiter (3), eine Schutzgasquelle und eine Spannungspulse erzeugende Spannungsquelle umfaßt, wobei die Wolfram-Nacktwendel einseitig im Reflektor gesockelt und mit der Spannungsquelle verbunden ist, der Lichtwellenleiter (3) in den halboffenen Reflektor hineinragt, so daß zwischen Lichtwellenleiter (3) und Reflektor eine Öffnung definiert wird, über die Schutzgas in den Reflektor ein- und/oder ableitbar ist.

11. Pulsbare NIR-Lichtquelle zur Erzeugung von Pulslängen größer 20ms, insbesondere zur Verwendung in einer Bestrahlungsanordnung nach einem der Ansprüche 1 bis 3, dadurch gekennzeichnet, daß die NIR-Lichtquelle (2) als Glühwendel-Halogenlampe ausgebildet ist, der eine Spannungspulse erzeugende Spannungsquelle zugeordnet ist, wobei die Spannungspulse einen Überlastbetrieb der Glühwendel-Halogenlampe erzeugen.

12. Pulsbare NIR-Lichtquelle nach Anspruch 11, dadurch gekennzeichnet, daß die Glühwendel (13) aus Wolfram gebildet ist.

13. Pulsbare NIR-Lichtquelle nach Anspruch 11 oder 12, dadurch gekennzeichnet, daß um den Quarzhüllkörper der Glühwendel-Halogenlampe teilweise ein Reflektor angeordnet ist.

**EP 1 038 505 A2**

**14.** Pulsbare NIR-Lichtquelle nach einem der Ansprüche 10 bis 13, dadurch gekennzeichnet, daß die strahlende Fläche durch chemische und/oder physikalische Verfahren mikrostrukturiert ist, so daß unerwünschte Wellenlängen interferenzoptisch ausgelöscht werden.

**Fig. 1**

Fig.2

**Fig.3**

1

13

17

1

5

9

3

7

6

8

11

14

Fig.4